# EUROPEAN PATENT APPLICATION

(11) **EP 0 624 612 A1**
(43) Date of publication of application: **17.11.1994**
(21) Application number: 94302893.6
(22) Date of filing: 22.04.1994
(51) Int. Cl.: C08G 18/62, C08G 18/65, C08G 18/72, A61L 29/00

(54) **Biostable polyurethane and medical article therefrom**

(30) Priority: 10.05.1993 US 59923
(71) Applicant: Becton Dickinson and Company, Franklin Lakes, New Jersey 07417-1880 (US)
(72) Inventor: Mann, Gregory J., West Jordan, Utah 84084 (US)
(74) Representative: Ruffles, Graham Keith

(57) **Abstract**

A melt processable segmented polyurethane with a hard segment composition in the range about 20 percent to about 40 percent. The polyurethane of the invention has a soft segment that includes an aliphatic diol having a molecular weight greater than about 1000. The aliphatic diol used in the invention is substantially free of ester and ether linkages, thus substantially eliminating potential sites for biodegradation and formation of potentially harmful low molecular weight extractable fragments. As a result of the low percentage hard segment, the polyurethane of the invention is inherently soft without the use of low molecular weight plasticizers and is well suited for applications requiring prolonged exposure to body fluids and enzymes. Such applications include pacemaker lead insulation, indwelling catheters, drainage devices and the like.

## Description

### 1. Field of the Invention

This invention relates to biomedical devices, and more particularly to a new polyurethane having resistance to degradation by body fluids suitable for forming or for coating medical devices intended for internal implantation.

### 2. Background of the Invention

In the biomedical art area, much research effort is expended to develop and qualify materials for implantation in the body. To be suitable for such applications, a material must not only have physical properties such as strength and flexibility, it should be stable to the various fluids and enzymes present in the body.

Many types of compounds are used in these applications, and polyurethanes often are selected. In general, polyurethanes have a good balance of physical and mechanical properties as well as superior blood compatibility when compared to other polymers such as silicone rubber, polyethylene, polyvinyl chloride and perfluorinated polymers. Some important applications of polyurethanes include peripheral and central venous catheters, coatings for pacemaker leads, drainage devices, implants and the like.

Polyurethanes are synthesized from three basic components; a diisocyanate, a polyglycol and a chain extender, usually a low molecular weight diol, diamine or water. If the extender is a diol, the polyurethane consists entirely of urethane linkages. If the extender is water, amino alcohol or a diamine, both urethane and urea linkages are present and the polyurethane is more accurately and conventionally termed a polyurethaneurea.

Polyurethanes develop microdomaines, conventionally termed hard segments (HS) and soft segments, and as a result are often referred to as segmented polyurethanes or block copolymers. The hard segments form by localization of the portions of the polymer molecules which include the diisocyanate and the extender components and are generally highly crystalline. The soft segments form from the polyglycol portions of the polymer chains and generally are either amorphous or only partially crystalline. A way of describing the difference between the hard and soft segments may be to consider the components comprising the hard section as a homopolymer having a glass transition temperature (T_{g}) above the normal use temperature range with the components comprising the soft segment as a homopolymer having a T_{g} below the normal use temperature. Thus, the bulk properties of the copolymer are a function of the degree of crystallinity and hard segment content. W. Lemm in Polyurethanes in Biomedical Engineering; edited by H. Planch, G. Ebiers and I. Syre; Elsevier Science Publishers (1984); pp 103-109 presents a discussion of polyurethane structure and its effects on biodegradation.

Typical polyglycols used for synthesis of polyurethanes are polyetherglycols and polyesterglycols. There is considerable literature documenting that polyurethanes having ester or ether linkages in their soft segment are subject to gradual degradation when exposed to the various fluids and enzymes present in the body. Additionally, since the hard segments or crystalline regions tend to be discrete regions embedded in a more continuous soft segment phase, particularly in low (30-50 percent) hard segment content formulations, it is believed that the soft section may be where much of the biodegradation occurs.

United States Patent No. 4,873,308 teaches that segmented polyurethanes which are substantially free of ester and ether linkages are particularly hydrolytically and oxidatively stable. The '308 patent teaches that "softer" segments of polyurethanes preferably consist of hydrocarbon backbones of fatty acid dimer derivatives alternating with short to medium chain hydrocarbon moieties and linked to them by urethane groups. The fatty acids, generally C₁₈, are dimerized and then formed into either diisocyanates or diol derivatives. The '308 patent further teaches that one or more short to medium chain length hydrocarbon moieties derived from diols or diisocyanate having a molecular weight of less than about 1000 may be incorporated into the "softer" segments. The use of these low molecular weight components in the soft segment results in some urethane linkages occuring in the soft segment, since there is a finite statistical probability that some of the diol will react on both its functional groups with a diisocyanate also reacted with a low molecular weight component. Further, since in any given mass of the dimer acid derived diol or diisocyanate the numbers of low molecular weight molecules constitute a substantial number of the molecules actually present, there is increased probability of occurrence of low degree of polymerization and low molecular weight molecules. Low molecular weight molecules of low degree of polymerization are more mobile, have larger numbers of potential biodegradation sites and may be more likely to be extractable into body fluids. The presence of urethane linkages in the soft segment may reduce the differentiation of hard and soft segments. Further, the presence of the urethane linkages in the soft segment makes them more accessible for hydrolysis than they would be in a more well defined crystalline hard segment embedded in a soft segment continuous phase.

While the above-referenced patent has provided improved materials for biomedical devices, further improvements, in particular polyurethanes having long term stability combined with strength and softness, are still needed. The present invention is directed to fulfilling this need.

### Summary of the Invention

A thermoplastic substantially hydrophobic polyurethane is formed from an aliphatic diol oligomer having a molecular weight greater than about 1000, a chain extender such as short chain diols, diamines, amino alcohols or water and a diisocyanate. Preferred polyurethanes include a nonaromatic or an aromatic diisocyanate or, in the alternative, mixtures of aromatic and nonaromatic diisocyanates in the hard segment. The soft segment includes an aliphatic diol having a molecular weight greater than about 1000, preferably formed by polymerization of butadiene. The preferred polyurethane has a hard segment between about 20 percent to about 40 percent by weight of the polymer and a soft segment substantially free of polyester and polyether bonds, thereby substantially eliminating reactive sites for hydrolysis and enzymatic attack.

Polyurethanes of the present invention are resistant to environmental biodegradation as evidenced by stress cracking induced by enzymes, fluids and ions present in a living body. The present polyurethane may be melt processed and has an ultimate tensile strength approaching that of comparably processed Teflon® polytetrafluoroethylene and Vialon® polyurethane. The tensile strength of the present polyurethane is about 50 percent greater than Kraton® elastomer or silicone polymers.

A further benefit of the polyurethane of the present invention is the substantial absence of extractables. Since the hard segment of the preferred polyurethane of the present invention is between about 20 percent to about 40 percent, the softness property is inherent, i.e., no plasticizers are required. Additionally, since the aliphatic diol used in the soft segment is substantially free of ester and ether bonds, which are sites for biodegradation, formation of potentially harmful low molecular weight fragments resulting from metabolic attack is substantially eliminated. The substantial elimination of these degradation products thus provides improved biocompatability over a polyurethane with ether and ester bonds in the soft segment.

### Brief Description of the Drawing

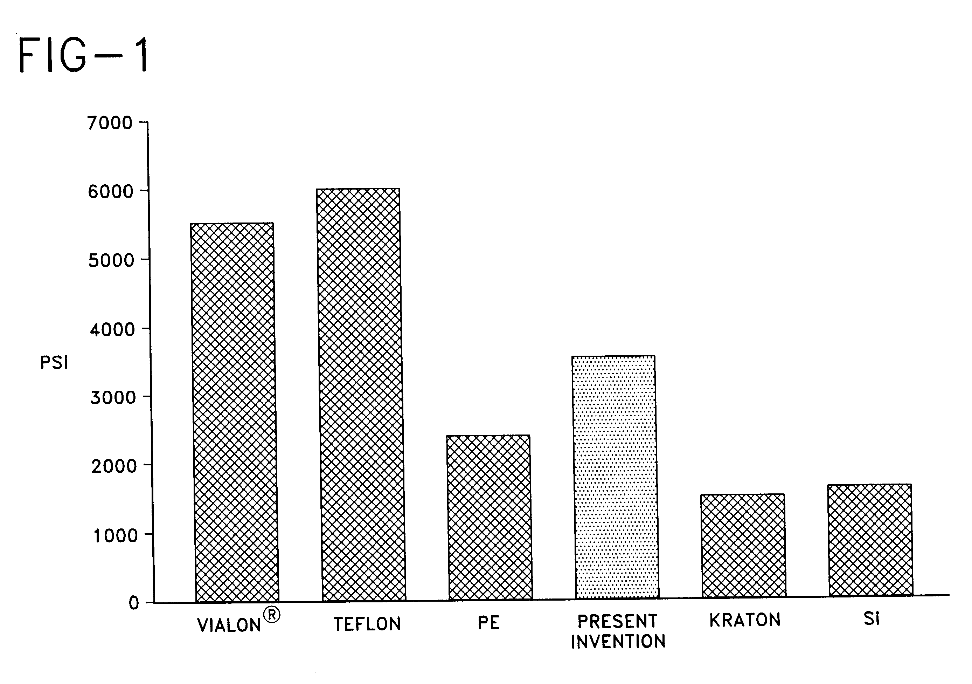

Fig. 1 is a graphical representation of the tensile strength of several elastomers as pressed films compared to the polyurethane of the present invention.

### Detailed Description of the Invention

While this invention is satisfied by embodiments in many different forms, there will herein be described in detail preferred embodiments of the invention, with the understanding that the present disclosure is to be considered as exemplary of the principles of the invention and is not intended to limit the invention to the embodiments described. The scope of the invention will be measured by the appended claims and their equivalents.

In accordance with the invention, the polyurethane of the present invention is substantially hydrophobic and has physical properties that make it useful for any application where water absorption is not desired.

The polyurethane of the invention may be prepared, as described below, from three essential components. The first component of the new polyurethane is the diisocyanate component. This component includes nonaromatic and aromatic diisocyanates. The nonaromatic diisocyanate may be any aliphatic or alicyclic diisocyanate as known in the polyurethane art. Suitable nonaromatic diisocyanates have from about 4 to 18 carbon atoms and may be branched or straight chain. Representative nonlimiting examples of such nonaramatic diisocyanates are 1,12-dodecanediisocyanate, 1,11-undecanediisocyanate, 1,10-decaneddisocyanate, 1,9-nonanediisocyanate, 1,8-octanediisocyanate 1,7-heptanediisocyanate, trimethyl 1,6-hexane diisocyanate, 1,4-cyclohexane diisocyanate, 4,4'-dicyclohexylmethyldiisocyanate and preferably 1,6-hexanediisocyanate (HDI). The aromatic diisocyanate may be any aromatic diisocyanate as known in the polyurethane art.
Representative nonlimiting examples of suitable aromatic diisocyanates are toluene diisocyanate, 3,3'-diphenylmethane diisocyanate, 1,4-phenylene diisocyanate, 2,2'-dimethyl-4,4'-biphenyldiisocyanate and 3.3'-dimethyl-4,4'-biphenyldiisocyanate. The preferred aromatic diisocyanate is 4,4'-diphenylmethane diisocyanate (MDI). Additionally, mixtures of nonaromatic and aromatic diisocyanates may be used. The ratio for mixtures of nonaromatic and aromatic diisocyanates may range between about 65:35 to about 35:65. For example, the ratio of HDI to MDI in the diisocyanate fraction may be between about 35:65 to about 65:35, respectively, and is preferably about 50:50 mole percent.

The second component of the new polyurethane is a chain extender. Suitable chain extenders may be low molecular weight branched or unbranched diols, diamines or amino alcohols up to ten carbon atoms, optionally fluorinated or mixtures thereof. Representative non-limiting examples of chain extenders are ethylene glycol; diethylene glycol; triethylene glycol; 1,2-propane diol; 1,3-propane diol; 1,6-hexanediol; 1,4-hydroxymethyl cyclohexane; ethanolamine; ethylenediamine; and hexamethylene diamine. Preferred chain extenders are 1,6 hexane diol, ethylenediamine, hexamethylenediamine and most preferably, 1,4-butane diol (BDO).

The third component of the new polyurethane is an aliphatic diol having a molecular weight greater than about 1000. The reaction of this aliphatic diol with the diisocyanate substantially forms the soft segment of the novel polyurethane. Representative non-limiting examples of suitable aliphatic diols are Polytail® H and Polytail® HA available from Ken Seika Corp. of Little Silver, N.J. These Polytail® aliphatic diols are exemplified by the structure: 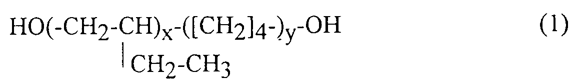
with x and y being integers.

The Polytail® aliphatic diols are prepared by the polymerization of butadiene followed by hydrogenation to substantially reduce carbon-carbon double bonds. The substantial reduction of carbon-carbon double bonds substantially reduces crosslinking during melt processing. It will be apparent to one skilled in the art of polymers that other polyolefins may be used as a source for the olefin diol. An example of an alternate polyolefin is hydroxy terminated polyisoprene, such as TH-21 as supplied by Ken Seika Corp., Little Silver, NJ. TH-21 has a molecular weight about 2600 and, since it is substantially difunctional, an equivalent weight of about 1300. The hydroxyl terminated-polyisoprene TH-21 has the structure 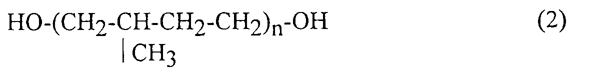
with n being an integer.

Polymers such as hydroxyl terminated polyisoprene may be hydrogenated to substantially reduce carbon-carbon double bonds. The use of hydroxyl terminated hydrogenated butadiene is preferred.

In the preparation of the preferred aliphatic diol from butadiene, two modes of polymerization are used, ionic and radical. If an ionic (anion) mode of polymerization is used, structure (1) has a higher proportion of pendent vinyl groups, with the x/y ratio ranging from about 7:1 to about 10:1 with a ratio of about 9:1 being preferred. This ionically polymerized diol is named Polytail® HA. Polytail® HA has a molecular weight about 2200 and since it is difunctional, an equivalent weight of about 1100. Alternatively, if the butadiene is polymerized using a radical mode of polymerization, structure (1) has a lower proportion of pendent vinyl groups and the x/y ratio will range from about 1:10 to about 4:6, a ratio of about 2:8 being preferred. The radically polymerized diol is named Polytail® H. Polytail® H has a molecular weight of about 2800 with an equivalent weight of about 1400. The resultant dihydroxy polybutadiene from either mode of polymerization is then catalytically hydrogenated to substantially reduce carbon-carbon double bonds. The hydrogenation results in a substantially saturated aliphatic hydroxyl terminated diol having less than 20% residual carbon-carbon double bonds. The presence of residual carbon-carbon double bonds may allow crosslinking during thermal processing. The most preferred aliphatic olefin will have the least amount of residual carbon-carbon double bonds.

The hard segment (HS) content of the polyurethane of the present invention may be about 10 percent to about 70 percent by weight. Desirably the present polyurethane HS is about 20 to about 40 percent, and is preferably about 30 percent. Many commercially important polyurethanes are gummy and tacky when formulated with HS below about 40 percent. The tackiness is believed to occur because the soft segment is more available at the surface with the hard segment occurring as discrete crystalline segments embedded in a continuous soft segment phase. For many of the commercially important polyurethanes, since there are polyester and polyether linkages present in the soft segment, the soft segment is believed to be where biodegradative attack occurs. The initial evidence of the biodegradation is often surface crazing and cracking. The crazing then developing into fissures allowing penetration into the bulk material.

In this description, the percentage HS is calculated based on the isocyanate fraction and extender (i.e., the weight of the chain extender plus the weight of the isocyanate fraction divided by the total weight of the reactants used times 100). An isocyanate index of 0.9 to about 1.2 may be used with an index of about 1.02, a slight excess, being preferred. Except in the case where water is used as a chain extender, reagents used in the preparation of the present polyurethanes should be substantially free from water. The preferred isocyanate index of 1.02 compensates for the presence of residual small amounts of water present in the reaction system.

The present polyurethane is preferably synthesized with aliphatic diol prepared from anionic polymerized butadiene or from radically polymerized butadiene. Alternatively a mixture the diols prepared by both methods may be used. Preferably, the mixture of diols is a mixture of diols including, but not limited to, about 40 percent to about 80 percent Polytail® HA having an x/y ratio of about 9/1. Example 7 demonstrates that a hydroxy terminated polyisoprene may also be used as the diol. The following examples are provided to further illustrate the invention but are not to be considered as limitative of the invention.

### Example 1

In a well-stirred reactor, 25.3g (0.56 equivalents, [eq.]) of butane diol (BDO) was mixed with 94.7g (0.76 eq.) of 4,4'-diphenylmethane diisocyanate (MDI) and 180g (0.76 eq.) aliphatic diol Polytail® H. The material formed was a stiff, hard polyurethane having a 40 percent (wt./wt.) hard segment which, when pressed into a film, exhibited signs of poor weld-together, having knit lines and discontinuities, was cloudy and had low elasticity. The polyurethane had ultimate tensile strength of about 2500 psi and an elongation at break of about 165 percent.

### Example 2

In a well-stirred reactor, 18.8g (0.42eq.) of BDO was mixed with 71.8g (0.569 eq.) of MDI and 210g (0.15 eq.) of aliphatic diol Polytail® H. The material formed was a stiff, hard polyurethane having a 30 percent (wt./wt.) hard segment, which when pressed into film exhibited signs of poor weld-together, having knit lines, was cloudy and had low elasticity. The polyurethane had ultimate tensile strength of about 900 psi and an elongation at break of about 100 percent.

### Example 3

In a well-stirred reactor, 14.3g (0.33eq.) of BDO was mixed with 60.2g (0.48 eq.) of MDI, 87.5g (0.063 eq.) aliphatic diol Polytail® H and 87.5g (0.08 eq.) aliphatic diol Polytail® HA. The material formed was a flexible polyurethane with a durometer of about 80A having a 30 percent (wt./wt.) hard segment. The polyurethane when pressed into a film at about 200° C. to about 220°C. gave a substantially continuous translucent film with a ultimate tensile strength about 1500 psi and an elongation at break of about 200 percent.

### Example 4

In a well-stirred reactor containing 400 ml of toluene, 5.9g (0.13eq.) of BDO) was mixed with 24.1g (0.19 eq.) MDI, 35g (0.02 eq.) of aliphatic diol Polytail® H and 35g (0.02 eq.) aliphatic diol Polytail® HA. The reaction was maintained at about 50°C. for about 16 hours. The resultant 30 percent (wt./wt.) hard segment polyurethane was cast at about 70°C. on a glass surface. The polyurethane was substantially continuous and substantially transparent with a ultimate tensile strength of about 3500 psi and an elongation at break of about 400 percent. The cast material could be hot pressed at about 210°C. The remaining material from this experiment gelled, dried and chipped for an extrusion trial.

### Example 5

In a well-stirred reactor containing 50g dimethyl acetamide (DMAC) and 450g toluene, 52.5g (0.04 eq.) aliphatic diol Polytail® H and 52.5 g (0.05 eq.) aliphatic diol Polytail ® HA were mixed with 36.1g (0.29 eq.) MDI for about 3 hours at about 50°C. To this mixture, 8.9g (0.20 eq.) BDO in 100g DMAC was added and the reaction was continued for about one additional hour at 50°C. An aliquot of the solution was removed and allowed to cool to room temperature, producing gellation. An aliquot was placed into acetone and a gummy white precipitate formed which, when hot pressed, was discontinuous and was cloudy. The product formed was a 30 percent (wt./wt.) hard segment polyurethane which was cast, dried and chipped for an extrusion trial.

### Example 6

The chipped polyurethanes from Examples 4 and 5 were combined for an extrusion feasibility trial using a 0.75 inch extruder. The 30 percent (wt./wt.) hard segment polyurethane exhibited signs of cold extrusion until extruder temperatures reached about 250°C. At about 250°C rod was extruded, demonstrating extrusion feasibility. The extruded material was also hot pressed into film which was substantially continuous and elastic.

### Example 7

In a well-stirred reactor, 11.93 g (0.26 eq.) BDO was mixed with 48.07g (0.38 eq.) MDI and 140.0 g (0.1 leq.) hydroxy terminated hydrogenated polyisoprene (TH-21 Ken Seika Corp.). The material formed was a flexible polyurethane with a durometer of 60A having a 30 percent (wt./wt.) hard section.

Adverting to Fig. 1, tensile strength of the polyurethane of the present invention of Example 5 is graphically compared to other elastomers commonly used for biomedical applications. The tensile strength was determined on hot pressed films. The results show that a film of the polyurethane of example 5 approaches that of a Vialon® polyurethane or Teflon® polytetrafluoroethylene and exceeds Kraton® elastomer and many silicone elastomers in tensile strength. The present polyurethane is not tacky or gummy and displays surprising longitudinal rigidity even at the preferred low (about 20 to 40 percent; wt./wt.) HS when compared to other commercially available polyurethanes.

The preferred present polyurethane, with a HS in the range of about 20 to about 40 percent (wt./wt.), has an inherent softness without the addition of low molecular weight plasticizer compounds. The absence of these low molecular weight plasticizer compounds substantially eliminates a source of extractables from the present polyurethane. Additionally, since there are substantially no sites for biodegradation in the soft segment, the aliphatic diol being substantially free of ether and ester linkages, the present polyurethane is substantially free of extractable biodegradation products present in other polyurethanes thus providing a benefit to the art of implantable polyurethanes. Additionally, unlike the prior art as represented by United States Patent No. 4,873,308 cited above, there are substantially no low molecular weight moieties resulting from low degree of polymerization of low molecular weight diol molecules. The physical properties and biostability of the present polyurethane render it suitable for use as an indwelling catheter, vascular access devices and other devices for adding or removing fluids from a patient's body. Additionally, the present polyurethane may be used as a covering for pacemaker leads and the like where there are requirements for biocompatability and softness. Further, since the present polyurethane can withstand melt processing techniques, it may be formed into tubing, rods or injection molded into other shapes. Tubing, rods or other devices formed from polyurethanes having ester or ether linkages in their soft sections may have a covering of the present biostable polyurethane applied by coextrusion, solvent based coating and the like, thereby retaining the structural properties of the base polyurethane while taking advantage of the biostability of the present invention.

## Claims

1. A thermoplastic substantially hydrophobic polyurethane having a hard segment ranging from about 20 percent to about 40 percent by weight comprising the reaction product of a diisocyanate, a diol chain extender and an aliphatic diol, said aliphatic diol having a molecular weight greater than about 1000, being substantially free of ether and ester bonds, said aliphatic diol having a structure 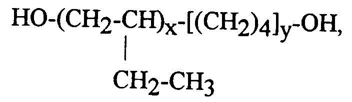 and wherein a ratio of x/y ranges between about 1:10 to about 10:1.

2. The aliphatic diol of claim 1 wherein said aliphatic diol is a mixture of diols comprising about 40 percent to about 80 percent of a first diol wherein x/y is about 9/1 and about 20 percent to about 60 percent of a second diol wherein x/y is about 2/8.

3. The polyurethane of claim 1 wherein said chain extender is a diol selected from the group consisting of triethylene glycol, 1,2 propane diol, 1,6 hexane diol, 1,12 dodecane diol, 1,4 hydroxymethylcyclohexane and 1,4 butane diol.

4. The polyurethane of claim 1 wherein said diol chain extender is 1,4 butane diol.

5. The polyurethane of claim 1 wherein said diisocyanate is an aromatic diisocyanate selected from the group consisting of 4,4'-diphenylmethanediisocyanate, toluene diisocyanate, 3,3'-diphenylmethane diisocyanate, 4,4'-biphenyl diisocyanate. 1,4-phenylene diisocyanate, 2,2'-diethyl-4,4'-biphenyl diisocyanate and 3,3'-dimethyl-4,4'biphenyl diisocyanate.

6. The polyurethane of claim 1 wherein said diisocyanate is 4,4'-diphenylmethane-diisocyanate.

7. The polyurethane of claim 1 wherein said diisocyanate is a nonaromatic diisocyanate selected from the group consisting of 1,12-dodecanediisocyanate, 1,11-undecanediisocyanate, 1,10-decanediisocyanate, 1,9-nonanediisocyanate, 1,8-octanediisocyanate, 1,7-heptanediisocyanateand trimethyl 1,6-hexanediisocyanate.

8. The polyurethane of claim 1 wherein said diisocyanate is a mixture of nonaromatic and aromatic diisocyanates selected from the group consisting of 4,4'-diphenylmethanediisocyanate, toluene diisocyanate, 3,3'-diphenylmethane diisocyanate, 4,4'-biphenyl diisocyanate, 1,4-phenylene diisocyanate, 2,2'-diethyl-4,4'-biphenyl diisocyanate, 3,3'-dimethyl-4,4'biphenyl diisocyanate, 1,12-dodecanediisocyanate, 1,11-undecanediisocyanate, 1,10-decanediisocyanate, 1,9-nonanediisocyanate, 1,8-octanediisocyanate, 1,7-heptanediisocyanate and trimethyl 1,6-hexanediisocyanate.

9. The polyurethane of claim 1 wherein said mixture of nonaromatic and aromatic diisocyanates has a ratio of nonaromatic to aromatic ranging between about 35:65 to about 65:35.

10. A catheter tubing comprising a thermoplastic substantially hydrophobic polyurethane, said polyurethane comprising the reaction product of an aromatic diisocyanate, a chain extender and an aliphatic diol, said aliphatic diol having a molecular weight greater than about 1000 and being substantially free of polyether and polyester bonds.
